# EUROPEAN PATENT SPECIFICATION

(11) **EP 2 526 981 B1**
(45) Date of publication and mention of the grant of the patent: **19.02.2014**
(21) Application number: 12169652.0
(22) Date of filing: 25.05.2012
(51) Int. Cl.: A61M 1/36

(54) **Infusion assembly of a dialysis machine**
Infusionsbaugruppe einer Dialysemaschine
Ensemble de perfusion d'une machine de dialyse

(30) Priority: 25.05.2011 IT BO20110302
(43) Date of publication of application: 28.11.2012
(73) Proprietor: Bellco S.r.l., Mirandola (IT)
(72) Inventor: Cianciavicchia, Domenico, 64040 Cavuccio (IT)
(74) Representative: Bergadano, Mirko

(56) References cited:
- EP-A2- 1 217 379
- WO-A1-2010/099816
- US-A1- 2003 225 371
- US-A1- 2004 133 145
- US-A1- 2005 234 382
- US-A1- 2007 235 083

## Description

The present invention relates to an infusion assembly of a dialysis machine.

As it is known, dialysis machines comprise infusion assemblies which are able to introduce substances into the liquids involved in dialysis treatment, whether this is blood, saline solution or plasma water.

Typical infusion assemblies of dialysis machines are described in the documents US 2004/133145 A1, US 2005/234382 A1, WO 2010/099816 A1 and EP 1 217 379 A2.

In particular, in extracorporeal dialysis treatments there is the need to introduce a first substance with anticoagulant action into the blood travelling along the arterial line in order to prevent the blood from coagulating during treatment. The introduction of such substance with anticoagulant action necessarily requires that the blood returning to the patient and, therefore, travelling along the venous line, is injected with a second substance which is able to contrast the anticoagulant effect of the first substance. As known to those skilled in the art, this need derives from the fact that it can be extremely dangerous to introduce blood characterized by a low coagulant capacity to the patient, in particular in patients suffering from specific diseases.

The substance that is normally used as anticoagulant is citrate saline solution, while the substance that contrasts the effects and which is introduced into the blood after the treatment is a solution of a calcium salt.

As may seem evident, it is extremely important to have an effective system to control the infusion of these substances, in order to prevent possible complications both to the treatment as a whole and to the patient's physical condition. In particular, it is extremely important to ensure control of the introduction of the substance which is able to restore the coagulation functions of the blood when it is returned to the patient.

Normally, the control system comprises a pressure measuring device arranged along the infusion line of the substance or a load cell arranged to measure the force exerted on a plunger of the syringe responsible for introduction of the substance.

However, these control systems have problems that in some circumstances could also compromise correct infusion of the substances.

In fact, the pressure measuring device arranged along the infusion line of the substance suffers from the problem of being unable to read any interruptions in flow that occur on the infusion line upstream of the sensor itself. In other words, if an interruption occurs on the syringe or on part of the infusion line between the syringe and the pressure measuring device, the pressure measuring device is unable to detect the presence of this interruption. In particular, one of the aforesaid interruptions can be caused by a clamp which has been applied erroneously to the infusion line.

Moreover, the load cell arranged to measure the action of the syringe plunger has low precision in relation to the effective quantity of substance infused.

Therefore, there is the need to provide an infusion assembly for a dialysis machine, whose technical characteristics are able to satisfy the requirements for control of the infusion and therefore to solve the problems of prior art.

The subject of the present invention is an infusion assembly of a dialysis machine, the essential characteristics of which are set forth in claim 1, and the preferred and/or auxiliary characteristics of which are set forth in claims 2 and 3.

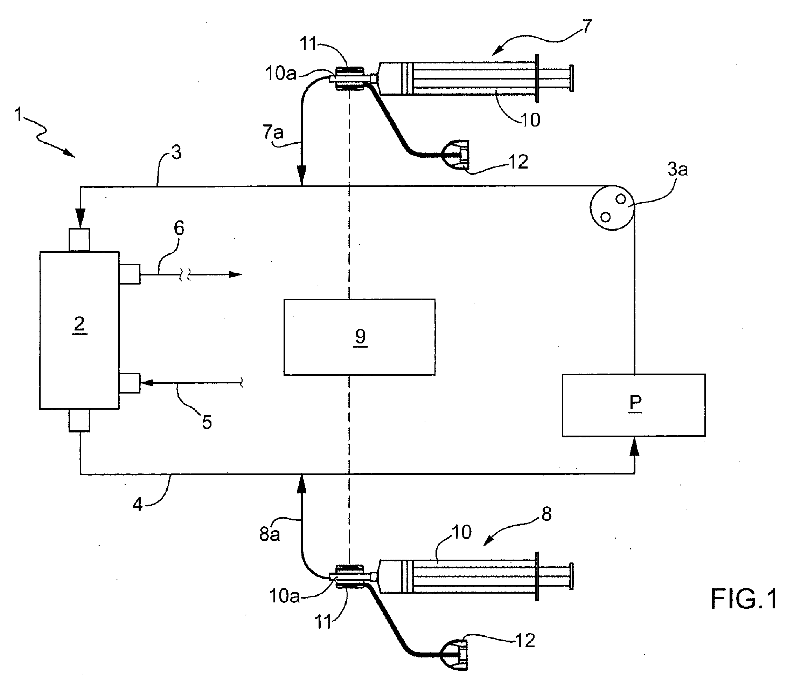

For better understanding of the invention there are set forth below embodiments provided purely by way of non-limiting example with the aid of the figure of the accompanying drawing, which schematically indicates an extracorporeal dialysis circuit with parts removed for clarity.

In the figure, the reference numeral 1 indicates as a whole and in schematic form an extracorporeal dialysis circuit according to the present invention.

The circuit 1 comprises a hemodiafiltration filter 2 (known and not described in detail), an arterial line 3 which is responsible for the transport of the blood from a patient P to the filter 2, a pump 3a applied to the arterial line 3 to ensure the movement of the blood, a venous line 4, which is responsible for the transport of the blood from the filter 2 to the patient P, an input branch 5 and an output branch 6 of the dialysing solution respectively into and from the filter 2, a first infusion assembly 7 responsible for the infusion of a substance with anticoagulant action into the blood present in the arterial line 3 and a second infusion assembly 8 responsible for the infusion into the blood present in the venous line 4 of a substance adapted to contrast the anticoagulant effect of the first substance.

Finally, the circuit 1 comprises a control unit 9 to which the two infusion assemblies 7 and 8 are connected.

Each of the two infusion assemblies 7 and 8 comprises a syringe 10 and a pressure sensor 11 arranged directly on a respective outlet nozzle 10a (known and not illustrated for simplicity). In particular, the infusion assemblies 7 and 8 comprise respective infusion lines 7a and 8a arranged to connect the syringes 10 respectively to the arterial line 3 and with the venous line 4.

As shown in the figure, each of the pressure sensors 11 comprises an electric connector 12 adapted to be connected to a monitor for visually checking the correct development of the infusions.

The presence of the pressure sensor 11 arranged directly on the outlet nozzle of the syringe or, in any case, connected to the outlet thereof, ensures effective control of correct operation of the syringe itself and, therefore, of the infusion as a whole. In fact, any interruption in flow caused by a malfunction of the syringe will be immediately felt by the pressure sensor 11. Moreover, the arrangement of the sensor directly on a outlet portion of the syringe definitively prevents the presence of a portion of infusion line upstream of the sensor and, therefore, the possibility of erroneous application of a clamp.

The circuit described above and illustrated in the figure refers merely by way of example to a hemodialysis treatment, while the invention described and claimed herein can be applied to any other dialysis treatment, such as a hemofiltration treatment or a hemodiafiltration treatment.

## Claims

1. An extracorporeal dialysis circuit (1) of a dialysis machine comprising at least one dialysis filter (2), an arterial line (3), which is responsible for the transport of the blood from a patient (P) to the filter (2), a venous line (4), which is responsible for the transport of the blood from the filter (2) to the patient (P), a first infusion assembly (7) responsible for the infusion of a first substance with anticoagulant action into said arterial line (3), and a second infusion assembly (8) responsible for the infusion into said venous line (4) of a second substance with such an action that contrasts the anticoagulant effect of the first substance; said first and said second infusion assembly respectively comprising a syringe (10) and an infusion line (7a, 8a) adapted to transport the substance injected respectively by the syringe (10) to the arterial line and by the syringe (10) to the venous line (4); said circuit being **characterised in that** both said infusion assemblies (7, 8) comprise a pressure sensor (11), which is directly connected to a respective outlet portion of said syringes (10).

2. The extracorporeal dialysis circuit according to claim 1, **characterised in that** said pressure sensor (11) comprises an electrical connector (12), which is adapted to be connected to a monitor for visually checking the correct development of the infusions.

3. A dialysis machine, **characterised in that** it comprises an extracorporeal dialysis circuit (1) according to any of the previous claims.

## Patentansprüche

1. Extrakorporaler Dialysekreislauf (1) einer Dialysemaschine mit mindestens einem Dialysefilter (2), einer arteriellen Leitung (3), die für den Transport des Bluts von einem Patienten (P) an den Filter (2) zuständig ist, einer venösen Leitung (4), die für den Transport des Bluts von dem Filter (2) an den Patienten (P) zuständig ist, einer ersten Infusionsanordnung (7), die für die Infusion einer ersten Substanz mit Antikoagulationsaktivität in die arterielle Leitung (3) zuständig ist, und einer zweiten Infusionsanordnung (8), die für die Infusion eines Stoffes in die venöse Leitung (4) zuständig ist, der dem Antikoagulationseffekt der ersten Substanz entgegenwirkt; die erste und zweite Infusionsanordnung weisen jeweils eine Spritze (10) und eine Infusionsleitung (7a, 8a) auf, die angepasst sind, um die Substanz zu transportieren, die von der Spritze (10) in die arterielle Leitung bzw. von der Spritze (10) in die venöse Leitung (4) injiziert wurde; der Kreislauf ist **dadurch gekennzeichnet**, das beide Infusionsanordnungen (7, 8) einen Drucksensor (11) aufweisen, der mit einem jeweiligen Auslassbereich der Spritzen (10) direkt verbunden ist.

2. Extrakorporaler Dialysekreislauf nach Anspruch 1, **dadurch gekennzeichnet, dass** der Drucksensor (11) einen elektrischen Verbinder (12) aufweist, der angepasst ist, um mit einem Monitor zum visuellen Überprüfen der richtigen Entwicklung der Infusionen verbunden zu sein.

3. Dialysemaschine, **dadurch gekennzeichnet, dass** sie einen extrakorporalen Dialysekreislauf (1) nach einem der vorstehenden Ansprüche aufweist.

## Revendications

1. Circuit de dialyse extracorporelle (1) d'une machine de dialyse comprenant au moins un filtre de dialyse (2), une ligne artérielle (3), qui est chargée de transporter le sang d'un patient (P) jusqu'au filtre (2), une ligne veineuse (4), qui est chargée de transporter le sang du filtre (2) jusqu'au patient (P), un premier ensemble de perfusion (7) chargé de la perfusion d'une première substance à action anticoagulante dans ladite ligne artérielle (3), et un deuxième ensemble de perfusion (8) chargé de la perfusion dans ladite ligne veineuse (4) d'une deuxième substance ayant une action telle qu'elle neutralise l'effet anticoagulant de la première substance ; lesdits premier et deuxième ensembles de perfusion respectivement comprenant une seringue (10) et une ligne de perfusion (7a, 8a) adaptée pour transporter la substance injectée respectivement par la seringue (10) jusqu'à la ligne artérielle et par la seringue (10) jusqu'à la ligne veineuse (4) ; ledit circuit étant **caractérisé en ce que** lesdits deux ensembles de perfusion (7, 8) comprennent un capteur de pression (11), qui est directement relié à une partie de sortie respective desdites seringues (10).

2. Circuit de dialyse extracorporelle selon la revendication 1, **caractérisé en ce que** ledit capteur de pression (11) comprend un connecteur électrique (12), qui est adapté pour être connecté à un dispositif de surveillance permettant de vérifier visuellement le développement correct des perfusions.

3. Machine de dialyse, **caractérisée en ce qu'**elle comprend un circuit de dialyse extracorporelle (1) selon l'une des revendications précédentes.
